(19)

**Europäisches Patentamt**
**European Patent Office**
**Office européen des brevets**

(11) **EP 1 965 868 B1**

(12) **EUROPEAN PATENT SPECIFICATION**

(45) Date of publication and mention
of the grant of the patent:
**19.09.2012 Bulletin 2012/38**

(51) Int Cl.:
*A61K 8/66* (2006.01)         *A61K 8/97* (2006.01)
*A61Q 11/00* (2006.01)

(21) Application number: **06801605.4**

(22) Date of filing: **16.08.2006**

(86) International application number:
**PCT/US2006/031953**

(87) International publication number:
**WO 2007/061468 (31.05.2007 Gazette 2007/22)**

(54) **NON-PEROXIDE PREPARATION FOR WHITENING NATURAL AND MANUFACTURED TEETH**

PEROXID-FREIE ZUBEREITUNG ZUR AUFHELLUNG VON NATÜRLICHEN UND KÜNSTLICHEN ZÄHNEN

PREPARATION DEPOURVUE DE PEROXYDE DESTINEE A BLANCHIR DES DENTS NATURELLES OU ARTIFICIELLES

(84) Designated Contracting States:
**AT BE BG CH CY CZ DE DK EE ES FI FR GB GR HU IE IS IT LI LT LU LV MC NL PL PT RO SE SI SK TR**

(30) Priority: **17.11.2005 US 281009**

(43) Date of publication of application:
**10.09.2008 Bulletin 2008/37**

(73) Proprietor: **Tom's of Maine**
**Kennebunk, ME 04043 (US)**

(72) Inventor: **BERGERON, Chantal**
**Kennebunkport, ME 04046 (US)**

(74) Representative: **Jenkins, Peter David et al**
**Page White & Farrer**
**Bedford House**
**John Street**
**London WC1N 2BF (GB)**

(56) References cited:
**EP-A- 1 050 295          EP-A- 1 941 862**
**WO-A-98/29088          CN-A- 1 107 308**
**US-A- 4 476 108          US-A1- 2002 122 775**
**US-A1- 2005 025 721     US-A1- 2005 210 615**
**US-B1- 6 331 291**

Remarks:
The file contains technical information submitted after the application was filed and not included in this specification

**EP 1 965 868 B1**

**Description**

BACKGROUND

Tooth whitening (*i.e.*, the removal of stains from teeth) is a multibillion-dollar industry world wide. Numerous approaches have been tried to whiten teeth, the two most common of which are the use of abrasives, such as diatomaceous earth, silica and baking soda, or chemical whiteners, such as peroxides. Abrasives in combination with a polishing action are used to polish discolorations and stains off of the surface of the teeth. Thus, light reflected from the teeth represents the true intrinsic color of the teeth. Abrasives are a major element of most consumer toothpastes and prophylaxis pastes used by dentists. Because abrasives only work on the surface of the teeth, the intrinsic color of the tooth is largely unchanged. As such, abrasives only offer limited effectiveness in whitening of the teeth and many abrasives used by prior art formulations can damage the enamel on the teeth because they contain or use harsh non-natural abrasives.

The second approach is the use of chemical whitening additives in a composition to intrinsically and extrinsically whiten teeth. Chemical whitening additives are applied to the teeth for period of time to allow the active ingredient to act upon the tooth and provide an improvement in the whiteness of the teeth. Whiteners are commonly applied to the teeth using toothpastes, rinses and mouthwashes, gums, floss, tablets, strips and trays. A common chemical whitening agent is peroxide. Often, strips and trays are used to apply peroxide for contact times beyond that achievable with typical tooth brushing. Concentration of the whitening active ingredient, contact time and number of applications are some of the primary parameters which dictate the rate and amount of whitening achieved with peroxide based tooth whitening compositions. Whitening products using peroxides are known in the art. For example, U.S. Pat. Nos. 5,891,453 and 5,879,691 describe a whitening product comprising a peroxide composition. However, peroxides can cause soft tissue (*e.g.*, gums and other oral tissues) irritation and some persons do not like to put harsh chemicals in their mouths. Also, both of these currently available approaches for whitening teeth are not effective for the whitening of manufactured teeth. U.S. Patent No. 6,331,249 discloses a dentifrice containing a peroxide composition as well as a protease, however, it still suffers from the same problem as the other chemical based prior art compositions in that it contains potentially damaging peroxides as well as other harsh chemicals.

Therefore, what is needed are compositions and methods for the whitening of both natural and manufactured teeth that do not utilize harsh abrasives or peroxide-based chemicals.

US-B1-6,331,291 discloses dentifrice gel/paste compositions.

SUMMARY OF INVENTION

The present invention provides a peroxide-free method according to claim 1 for whitening natural or manufactured teeth, and a peroxide-free product according to claim 6. Preferred features are defined in the dependent claims.

The present invention relates to methods for the whitening of natural and manufactured teeth without the use of peroxides or abrasives. The active ingredient in the present invention is "actinidin" (crude, partially purified or purified) and is produced from kiwifruit. The present invention provides methods using actividin as a tooth-whitening agent in, for example, a strip, tray, dentifrice (*e.g.*, toothpastes and tooth powders), dentures cleaner, mouth rinse, mouthwash or chewing gum .

DESCRIPTION OF FIGURES

Figure 1 shows the effect of the percentage of glycerin on the color change of hydroxyapatite disks treated with a 5 % solution of crude actinidin colored with tea and coffee.

Figure 2 shows the effect of the time on the color change of hydroxyapatite disks treated with a 5 % solution of crude actinidin colored with tea and coffee.

Figure 3 shows the casein hydrolysis assay dilution map.

Figure 4 shows the casein hydrolysis assay final assay map.

DESCRIPTION OF THE INVENTION

The present invention relates to a non-abrasive or natural abrasive oral composition for whitening natural teeth, crowns and other manufactured teeth, without the presence of peroxide or abrasives, to restore the natural color of the teeth. More specifically, the invention relates to the use of a botanical ingredient (crude or purified actinidin) as whitening agent in a strip or tray (*See,* for example, U.S. Patent Nos.: 6,949,240, 6,514,483 and 5,702,251 as well as the U.S. Patents cited therein), dentifrice (*e.g.*, toothpaste, denture cleaning agent or toothpowder, *See,* for example, U.S. Patent Nos.: 6.861.048: 6.555.094, as well as the U.S. Patents cited therein), rinse and mouthwash (*See,* for example, U.S.

Patent No.: 6,004,538, as well as the U.S. Patents cited therein) or chewing gum (*See*, for example, U.S. Patent Nos.: 6,926,916, 6,416,774 and 4,146,634, as well as the U.S. patents cited therein), all of which the manufacture is known in the art. In some embodiments, glycerin is added to the actinidin composition in the range of 5 % to 100 % of the total composition. Specific embodiments of the invention are described in greater detail below.

The botanical ingredient referred to alternatively as "crude actinidin" or "actinidin-containing preparation" and is produced from kiwifruit. Experimental work has demonstrated the effectiveness of formulations of substantially pure actinindin in the whitening of teeth, as well as the effectiveness of formulations of a crude actinidin preparation having a defmed minimum actinidin specific activity. The production process used to generate crude actinidin removes the skin and seeds from kiwifruit pulp without using chemicals, for example, and the crude product may then be freeze-dried for storage. Actinidin (EC 3.4.22.14), also known as actinidain, is a kiwi protease. The crude actinidin has a minimum activity of about 3,500 U/g (units per gram) and a typical activity of about 5,000 U/g assessed by a casein hydrolysis assay method as described in detail in the Experimental section of this specification.

As disclosed herein, "purified actinidin" or "partially purified actinidin" is actinidin substantially purified from crude actinidin by protein purification procedures known in the art *(See,* for example, Molecular Clowning: A Laboratory Manual, 2nd Ed., eds. Sambrook, Fritsch and Maniatis, Cold Spring Harbor Press, Cold Spring Harbor, N.Y., 1989 and Protein Purification Protocols, 2nd Ed., ed. Culter, SmithKline Beecham Pharmaceuticals, Harlow, UK, 2003). Purified or partially purified Actinidin has an acitivity as assayed by a casein hydrolysis assay method (as described in the Experimental section of this specification) in the range of 20,000 U/g to 100,000 U/g. Protein purification techniques known in the art include, for example, protein precipitation (with, for example, salts or polymers), immuno-affinity chromatography, affinity chromatography, buffer exchanges and diffusion (to remove, for example, ionic impurities), ionic exchange chromatography, ultrafiltration, microfiltration, hydrophobic interaction chromatography, hydrophobic interaction chromatography, size-exclusion chromatography, electrophoresis, etc. In one embodiment of the present invention, purified actinidin is at least 50 % pure. In a more preferred embodiment, purified actinidin is at least 70 % pure. In an even more preferred embodiment, purified actinidin is at least 90 % pure. All of the methods and compositions of the present invention are compatible with both crude and purified actinidin.

Purified, partially purified or crude actinidin-containing preparations are formulated for application to natural or manufactured teeth at a concentration range of about 50 to 1200 U/g. In a more preferred embodiment, the crude actinidin is used in compositions of the present invention at a concentration range of about 100 to 1000 U/g as determined by the casein hydrolysis assay (as described in the Experimental section). The crude actinidin preparation described above, which is prepared by removing skin and seed, followed by freeze-drying, is used in the compositions of the present invention at a concentration range of about 1 to 12 % w/w of the final composition. In a more preferred embodiment, crude actinidin is used at a concentration range of about 2 to 10 % w/w of the final composition. If purified (or partially purified) actinidin is used it is used at the same final concentration as measure by U/g as is the crude actinidin.

The actinidin-containing preparation, when formulated for application to natural or manufactured teeth, is effective for the remove the stains on the surface of the tooth (extrinsic stains) and below the surface of the tooth (intrinsic stains) caused by polyphenol-containing foods like tea, coffee, blueberry or wine and can be used in a oral composition to whiten and restore the natural color of natural and manufactured teeth. In the instant application "manufactured teeth" (or "manufactured tooth") refers to dentures, crowns, bridges, partials or any other dental appliance designed to replace either totally or partially a natural tooth or teeth. The active ingredient of the present invention also prevents future stains as it slows down the build up of layers formed by saliva proteins (*i.e.*, pellicle), to which stains and stain causing agents adhere. In this regard, the actinidin-containing preparation is an anti-adherent that can prevent the buildup of plaque and prevent also the stains that adhere to plaque, tartar or pellicle.

Actinidin is a cysteine proteinase from the genus *Actinidia* (kiwifruit or also referred to as Chinese gooseberry). Actinidin contains a free sulfhydryl group, which is essential for the activity, and is, therefore, grouped in the class of plant thiol proteases. This group also includes papain from papaya, ficin from figs and bromelain from pineapple. Cysteine proteases can break down proteins. In the prior art, actinidin is used either as a digestive enzyme (as discussed in the next paragraph) or high quality meat tenderizer (King, et al., Meat Science, 5:389-396, 1981; Lewis, et al., J. Food Biochem, 12:147-158, 1988; Wada, et al., Food Chemistry, 78:167-171, 2002; Kowlessur, et al., Biochem. J, 259: 443-452, 1989). Other proteases in the papain family are used as dough conditioners, anti-inflammatory agents or in the brewing industry to prevent storage "chill haze."

Kiwifruit is known worldwide as a green-fleshed fruit. Kiwifruit is grown commercially in New Zealand, Chile, California and other geographic areas for a wide range of applications from juices and concentrates to wine production. Kiwifruit is the most nutrient-dense fruit in the world (Lachance, J Am Coll Nutr, 16:5, 1997) containing twice the vitamin C of an orange, the potassium of a banana, high contents of vitamin E, carotenoids, folic acids, manganese, and soluble and insoluble fibers and is low in sodium. Kiwifruit is also renowned for its enzymes, which are effective digestive regulators. Actinidin, which is the prominent kiwi enzyme, makes up 60 % of the total soluble protein in kiwifruit pulp (including skin and seeds) is used as a natural indigestion remedy. Unlike most conventional indigestion remedies, actinidin boosts the enzyme activity of the stomach rather than altering the pH.

Some cysteine proteases are used in oral care preparations, like papain as an anti-stain agent in certain toothpastes alone or in combination with other agents, and bromelain as anti-inflammatory agent In the absence of hydrogen peroxide, Actinidin (crude or purified) is not known for use as whitening agent for natural and manufactured teeth.

Some individuals appear to be more susceptible to extrinsic tooth staining than others, but the reasons for such differences are unclear (Watts and Addy, Food Chemistry 78:167-171, 2001). The extrinsic stains are adsorbed onto tooth surface deposits such as plaque or the acquired pellicle. The pellicle is a clear, thin layer of protein to which food, bacteria and stains attach. Pellicle is caused when specific salivary proteins bind to teeth and hydroxyapatite, the main mineral component of teeth (Nathoo, J Am Dent Assoc 128:6S-10S, 1997). Saliva contains a high proportion (up to 70 %; Kauffman and Keller, Arch Oral Biol., 24:249-256, 1979) of proline-rich proteins, which form pellicle. The proline-rich proteins of the pellicle, particularly basic proline-rich proteins, have a particularly high affinity for dietary polyphenols, which are believed to be at least partly responsible for dental staining (Hagerman and Butler, J Biol Chem, 256:494-4497, 1981; Proctor, et al., J Dental Research, 84:73-78, 2005).

The possible aetiological agents for extrinsic stains include dietary components, beverages, tobacco, mouthrinses and other medicaments as well as oral hygiene (Watts and Addy, Food Chemistry 78:167-171, 2001). Tea, coffee, red wine, and other foods rich in polyphenols are thought to be responsible for dietary tooth staining. Polyphenols are a diverse group of substances commonly found in plants. They range from small flavonoids, such as the green tea catechins and grape-skin anthocyanins, to highly polymerized structures containing more than 50 flavanol molecules, such as the black tea theaflavins and thearubigins (Bravo, Nutrit Rev, 56:317-333, 1999; Bennick, Crit Rev Oral Biol Med, 13:184-196, 2002). Black tea and red wine components have been shown to have a profound effect on *in vitro* pellicle maturation, causing thickened layers of stained material to build up, which are not readily removed (Joiner, et al., Eur J Oral Sci., 111:417-22, 2003). Bacteria and stains attach to the protein pellicle to form plaque. Although the present invention is not limited by any particular mechanism, the inclusion of a protease-containing kiwi extract in an oral composition is thought to contribute to the breakdown of the protein pellicle without abrasion and peroxides thereby removing stains and whitening the teeth.

Intrinsic stains are stains that are found below the surface of the tooth. The agents that cause these stains either lodge in pores in the tooth, bind to minerals in the tooth or bind to saliva proteins that have lodged below the surface of the tooth. The compositions and methods of the present invention are also effective at the lessening and removal of intrinsic stains thereby providing even greater whitening of both natural and manufactured teeth over prior art agents (*e.g.*, abrasives) that only are active against extrinsic stains.

Following is disclosure relating to specific embodiments of the present invention.

Whitening Strips

The manufacture of whitening strips and whitening trays are well known in the art. Briefly, for example, whitening strips may be made as follows. The strip may be a water hydratable film made out of, for example, poly(ethylene) oxide films in thickness of about 20 to about 1500 micrometer ($\mu$m) and preferably about 50 to about 1000 $\mu$m. The dried film contains the actinidin whitening agent in an inactive state. Hydration of the film by saliva in the oral cavity solubilizes the water soluble whitening agent incorporated in the exemplary ethylene oxide polymer matrix whereby the whitening agent is activated and released to the tooth surfaces to which the film is applied. In another embodiment, the film is pre-hydrated and the actinidin-based whitening agent is in a hydrated form. For dried strips, the rate at which the whitening agent is solubilized and thereafter released into contact with tooth surfaces is controlled by varying the film thickness, polymer properties, as well as the whitening agent concentration, such concentration generally varying from about 0.1 to about 30 % by weight and preferably about 0.5 to about 25 % by weight of the film.

Ethylene oxide polymers useful for purposes of the present invention include homopolymers or mixtures of ethylene oxide polymers of varying molecular weight ranging from about 10,000 Daltons and up to about 10,000,000 Daltons and preferably in the range of about 100,000 to about 1,500,000 Daltons. Such ethylene oxide polymers are commercially available from various sources. Poly(ethylene) oxide in the molecular weight range of 10,000 to 1,000,000 Daltons is available from the Union Carbide Company under the tradename "Polyox" and are preferred for purposes of the present invention. The ethylene oxide polymer comprises about 50 to about 95 % by weight of the film of the present invention and preferably about 60 to about 85 % by weight.

The plasticizer useful for purposes of the present invention are selected from glycols such as propylene glycol, polyethylene glycol, polyhydric alcohols such as glycerin and sorbitol and glycerol esters such as glycerol triacetate. The plasticizer comprises 5 to 30 % by weight of the film of the present invention and preferably about 10 to about 25 % by weight.

Glycerin is the preferred plasticizer for use in the present invention as well as propylene glycol or polyethylene glycol such as is available from Union Carbide Corporation as their series of Carbowaxes, which range in molecular weight from 200 to 600 Daltons.

In addition to the incorporation of actinidin-based whitening agents and plasticizer there may also be included in the film matrix or whitening agent minor amounts, *e.g.*, 0.01 to 2 % by weight of other natural ingredients such as antioxidants, preservatives, pH adjusting agents, desensitizing agents, stabilizing agents, gelling agents, flavors, etc. A preferred gelling agent is carrageenan. Preferred thickening agents are hectorite clay, propylene glycol and glycerin. Preferred flavorings are natural flavorings such as peppermint, spearmint, apricot and cinnamon, etc. For sensitive teeth, the formulation may contain an agent for relieving pain in people with sensitive teeth. A preferred ingredient for this purpose is potassium nitrate. Agents may be added to promote a desired mouth feel. A preferred ingredient for this is propolis. Cleansing agents may be used in the present invention. A preferred cleansing agent is sodium lauryl sulfate. Moisture retaining agents may be used in the present formulations. A preferred moisture retaining agents are sorbitol and xylitol.

One side of the ethylene oxide polymer film can be also coated with a thin protective coating layer, *e.g.*, of 10 nanometers (nm) to 500 microns ($\mu$) thickness which serves as a barrier to prevent release of the solubilized whitening agent from the matrix to the oral mucosa, thereby achieving unidirectional release of the whitening agent solely on the tooth surfaces.

The coating material is applied in a sufficiently thin layer so as not to interfere with the flexibility of the film and to allow the whitening strip to conform to an arrangement of a row of teeth.

The coating materials can be one or a combination of high molecular weight (that is, molecular weights greater than 1,000,000 Dalton) and include, for example, ethyl cellulose, propyl cellulose, isopropyl cellulose, butyl cellulose, t-butyl cellulose, cellulose acetate, and derivatives of polyvinyl alcohol such as polyvinyl acetate and shellac.

The ethylene oxide polymer film of the present invention can be prepared using conventional extrusion or solvent casting processes. For example, to prepare a film by solvent casting poly(ethylene) oxide, the ethylene oxide polymer or mixture of polymers is dissolved in a sufficient amount of a solvent which is compatible with the polymer. Examples of suitable solvents include water, alcohols, acetone, ethyl acetate or mixtures thereof. After a solution has been formed, a plasticizer is added with stirring, and heat is applied if necessary to aid dissolution, until a clear and homogeneous solution has been formed, followed by the addition of the whitening agent and any other ingredients such as flavors. The solution is coated onto a suitable carrier material and dried to form a film. The carrier material must have a surface tension, which allows the polymer solution to spread evenly across the intended carrier width without soaking in to form a destructive bond between the two substrates. Examples of suitable carrier materials include glass, stainless steel, teflon, polyethylene-impregnated kraft paper.

The film used in the instant invention may contain a wax but preferably does not contain a wax, ethylene terephthalate and/or polyethylene terephthate, polyethylene, Teflon™, ethylvinyl acetate, ethylenevinyl alcohol, polyesters, hydroxypropyl cellulose, polystyrene, polyurethane, and/or an antimicrobial agent.

Drying of the film, if desired, may be carried out in a high-temperature air-bath using a drying oven, drying tunnel, vacuum drier, or any other suitable drying equipment, which does not-adversely affect the active ingredient(s) or flavor of the film.

For ease of use, the dry film is cut into pieces of suitable size and shape and packed into a suitable container.

To use the whitening film strip of the present invention, the film when applied to the teeth surface when hydrated by saliva, if necessary, in the oral cavity or prewetted by dipping the strip in water will adhere to the teeth in an appropriate manner. In this regard, the whitening strip is formed to have a width dimension suitable to cover a row of teeth (upper or lower). Therefore, the whitening strip may be applied to the upper set of teeth, or to the lower set of teeth either separately or simultaneously. The length dimension of the whitening strip is determined by the amount of coverage desired. In this regard, the number of teeth, which it is desired to whiten will determine the dimensions of the whitening strip. For instance, it may be desired to only whiten the front teeth, which are most easily seen by others. Accordingly, the length of whitening strip can be reduced in this case, as compared to the case where it is desired to whiten all of the teeth. The duration of application of whitening strip to the teeth will depend upon the type and concentration of the whitening agent, as well as the type and intensity of stain.

In another embodiment, the strip of material may be formed from materials such as polymers, natural and synthetic wovens, non-wovens, foil, paper, rubber, and combinations thereof. The strip may also comprise a release liner. The strip of material (as well as the release liner) may be a single layer of material or a laminate of more than one layer. Suitable polymers include, but are not limited to, ethylvinylacetate, ethylvinyl alcohol, polyesters such as MYLAR® manufactured by DuPont, and combinations thereof.

The release liner can be formed from any material that exhibits less affinity for the tooth whitening composition than the tooth whitening composition exhibits for itself and for the strip of material. For example, the release liner can be formed from paper or a polyester, such as SCOTCHPAK® which is manufactured by the 3M Corp. of Minneapolis, Minn., which are coated with a non-stick material in order to aid release of the tooth whitening composition from the release liner when the strip of material is pulled away from the release liner. Exemplary coatings can include wax, silicone, fluoropolymers such as Teflon®, fluorosilicones, or other non-stick type materials. Also, suitable coatings might include one of the coatings described in U.S. Pat. Nos. 3,810,874; 4,472,480; 4,567,073; 4,614,667; 4,830,910; and 5,306,758. A further description of materials suitable which might be suitable as release agents is found in Kirk-Othmer Encyclopedia of Chemical Technology, Fourth Edition, Volume 21, pp. 207-218, incorporated herein by reference. The release liner should be at least the same size and shape as the whitening strip. However, the release liner can extend beyond the strip of material so that it is easier to remove the strip of material and the thin layer of actinidin-based whitening agent from the release liner.

While the actinidin-based tooth whitening composition is described herein as comprising, for example, both the strip of material and the release liner, it is contemplated that the actinidin-based tooth whitening composition may comprise only the strip of material and the thin layer of the actinidin-based tooth whitening composition. For example, the interior of a package storing the strip of material and the thin layer of the actinidin-based tooth whitening composition might be coated in a manner similar to that described above with respect to the release liner to facilitate removal of the strip of material and the thin layer of the actinidin-based tooth whitening composition from the package during use. Further, it is contemplated that the actinidin-based tooth whitening composition could be provided in the form of a roll rather than planar as described above and could comprise a plurality of strip of materials and/or release liners. Alternatively, it is contemplated that the strip of material and/or release liner might include other non-planar shapes such as preformed dental trays or flexible dental trays. The strip of material and/or release liner can also be formed from permanently deformable strips of material, wax, or any other material suitable for use as a barrier for the tooth whitening composition and for applying the tooth whitening composition to the teeth.

While the above-described materials for the strip of material and release liner are suitable for use with the present invention, the stability of the thin layer of the actinidin-based tooth whitening composition can be improved when the release liner and/or the strip of material (or at least the surfaces in contact with the actinidin-based tooth whitening composition) are formed from a polyolefin and, preferably, from polyethylene or polypropylene. Even small to moderate increases in the stability of a actinidin-based tooth whitening composition can have a significant impact on the shelf life of the product. As used herein, the term "stability" is intended to refer to the propensity of a actinidin-based tooth whitening composition to maintain at least 90 % of its original concentration or activity over a specified period of time (e.g., 3 months, 6 months, 12 months), wherein the specified period of time is measured beginning from the point at which the tooth whitening composition is manufactured and formed as a thin layer. Other polyolefin blends, polyethylene blends, polypropylene blends, and combinations thereof would also be suitable for use as the strip of material and/or the release liner in the present invention. As discussed above, the release liner can also be coated to aid release of the tooth whitening composition from the release liner during manufacture and/or use. However, these coatings generally do not act as barriers between the actinidin-based tooth whitening composition and underlying material such that proper selection of the underlying material is still desirable. Any coating should be inert, however, relative to the actinidin-based tooth whitening composition.

The strip of material and/or release liner are generally less than about 1 mm thick, preferably less than about 0.05 mm thick, and more preferably from about 0.001 to about 0.03 mm thick. Still more preferably, the strip of material and/or release liner are less than about 0.1 mm thick and yet more preferably from about 0.005 to about 0.02 mm thick. The thickness and the permeability of the strip of material and/or release liner may have an effect on the stability of the tooth whitening composition. In general, a thicker strip may provide more stability for the tooth whitening composition. However, the thickness of the strip of material must be balanced with the consumer acceptance of comfort of wearing the strip.

The strip of material should have a relatively low flexural stiffness so as to enable it to drape over the contoured surfaces of the teeth with very little force being exerted; that is, conformity to the curvature of the wearer's mouth, teeth, and gaps between teeth is maintained because there is little residual force within the strip of material to cause it to return to its substantially flat shape. The flexibility of the strip of material enables it to contact adjoining soft tissue over an extended period of time without physical irritation. The strip of material does not require pressure to form it against the teeth and it is readily conformable to the tooth surfaces and the interstitial tooth spaces without permanent deformation when it is applied.

Flexural stiffness is a material property that is a function of a combination of strip thickness, width, and material modulus of elasticity. This test is a method for measuring the rigidity of polyolefin film and sheeting. It determines the resistance to flexure of a sample by using a strain gauge affixed to the end of a horizontal beam. The opposite end of the beam presses across a strip of the sample to force a portion of the strip into a vertical groove in a horizontal platform upon which the sample rests. A microammeter, wired to the strain gauge is calibrated in grams of deflection force. The rigidity of the sample is read directly from the microammeter and expressed as grams per centimeter of sample strip width. In a preferred embodiment but not required for the present invention, the flexible strip of material has a flexural stiffness of less than about 5 grams/cm as measured on a Handle-O-Meter, model #211-300, available from Thwing-Albert Instrument Co. of Philadelphia, Pa., as per test method ASTM D2923-95. Preferably, the strip of material has a flexural stiffness less than about 4 grams/cm, more preferably less than about 3 grams/cm, and most preferably from about 0.1 grams/cm to about 1 grams/cm.

Whitening trays

Similar to whitening strips, whitening trays may be used to hold the actinidin-based whitening composition of the present invention close to the teeth.

A tray constructed in accordance with the present invention may be used for applying the actinidin-based whitening composition to teeth. The tray, for example, generally comprises an arch configured U-shaped trough, having a bottom wall and a somewhat arch-shaped exterior wall integral therewith and a somewhat arch-shaped interior wall, also integral therewith. It can be seen that the two walls are spaced from each other to form an interior channel or groove or so-called "trough" sized receive the teeth of the user.

The tray of the present invention is preferably formed of any of a number of plastic materials that are capable of being molded when heated. A vinyl plastic is one of the preferred materials since it can be heated and becomes pliable and moldable at a relatively low temperature, although the invention is clearly not limited to the use of vinyl plastics. Other plastic materials, such as styrene copolymers and the like, could also be used.

The tray may, but need not, be provided on its exterior surface with a handle for engagement by the user. This handle is preferably integral with the exterior wall and would extend outwardly between the lips of the user when the arch is disposed about the teeth of the user.

The upper edge of the exterior wall may be scalloped, that is, provided with a somewhat serpentine edge portion, as shown. In this way, the outer wall will more fully, conform to the roof portion of the mouth existing between the teeth and the lips of the user.

An arch-shaped insert is also located in the channel and essentially has an arch shape similar to that of the exterior wall. Moreover, the insert is slightly spaced inwardly from the interior surface of the exterior wall so as to form a reservoir. In this exemplary embodiment of the invention, the insert does not extend for the full distance of the exterior wall but does extend around a substantial portion of the arch to particularly span those teeth that are visible during a smile or conversation of a user.

The insert may be removably held in position to form the reservoir by means of tabs formed along the lower edge of the insert and which extend into detents or depressions formed on the interior surface of the exterior wall. However, any means for temporarily retaining the insert in the position in the tray may be used.

In one embodiment, the tray is molded to the user's teeth. For example, in order to form the tray and effectively mold the same to conform to the shape of the user's arch and teeth, the plastic forming the tray, and the liner is heated so as to become somewhat flexible and pliable, that is effectively moldable. Heating may be accomplished in any of a variety of ways. A typically convenient way is to insert the tray into a bath of hot water. The tray can be merely dipped into the water by holding the outer end of the handle. Thereafter, the tray is placed on the teeth of the user and allowed to effectively conform to the shape of the user's teeth. A simple tapping or pressing on the inner and outer surfaces of the tray and particularly the interior and exterior walls, will cause the tray to effectively conform to the shape of the user's teeth and arch.

After the tray has been removed from the user's mouth, it is again heated, typically by the same means, for example, introducing the same into a bath of hot water momentarily. The tray is then allowed to cool. In its cooled configuration, the tray effectively is a mirror image of the teeth and both the inner and outer surfaces thereof. Thus, the tray constitutes an effective mold of the user's teeth. Generally, one tray would be used for the upper teeth and another tray would be used for the lower set of teeth.

After the tray has been cooled, the insert is removed. Thus, there is an additional space between the front surface of the teeth and the interior surface of the exterior wall and that space is effectively the reservoir. The insert is of a relatively thin cross-sectional shape and typically is of the order of one-half millimeter in thickness. However, any reasonably sized insert may be used for this purpose. Moreover, the insert is preferably made of the same material used in the formation of the tray.

In another embodiment, the tray is pre-formed with a shape suitable for the application of the actinidin-based whitening composition to the user's teeth.

The actinidin-based whitening composition may be used in the whitening operation by applying the actinidin-based whitening composition in the tray and applying the tray to the user's teeth for a period of time and/or a number of applications necessary to achieve the desired whitening result. The actinidin-based formulation of the present invention when used in a whitening tray may also include additional ingredients as listed under "Whitening strips" above.

Dentrifrice

The actinidin-based whitening composition of the present invention may also be used when formulated with toothpastes and tooth powders.

Compositions in the form of toothpastes, denture cleansing liquids and pastes, and the like, will generally comprise a binder or thickening agent. Binders suitable for use herein include carboxyvinyl polymers, carrageenan (preferred), hydroxyethyl cellulose and water-soluble salts of cellulose ethers such as sodium carboxymethyl cellulose and sodium carboxymethyl hydroxyethyl cellulose. Natural gums such as gum karaya, xanthan gum, gum arabic, and gum tragacanth can also be used. Colloidal magnesium aluminium silicate or finely divided hydrated silica can be used as part of the thickening agent to further improve texture. Binders/thickening agents can be used in an amount from about 0.1% to about 5.0%, preferably from about 0.1 to about 1% by weight of the total composition. The actives are fluoride and potassium nitrate, their level are described in their respective FDA monographs for anti-cavity and sensitivity. Active for gingivitis are still pending the finalization of the monograph. The abrasives are calcium carbonate, baking soda or hydrated silica.

Such compositions will typically comprise one or more sweeteners and flavorings. Examples of suitable sweeteners and flavorings for use in dentrifrices are discussed above pH balancing agents as well as, surfactants, preservatives, stabilizers, etc., are also often used in dentrifrice compositions, examples of which are also provided above.

It is also desirable to include some humectant material in a toothpaste to keep the composition from hardening upon exposure to air. A preferred humectant is carrageenan. Certain humectants can also impart a desirable sweetness to toothpaste compositions. Liquid dentifrice and mouthwashes can also contain a quantity of humectant. Suitable humectants are well known in the art. When present, humectants generally represent from about 10 % to about 70 %, by weight of the compositions of the invention.

Toothpowders may make use of many of the same ingredients as toothpastes except that they must be mixed in a dried state (*i.e.*, dry milled) or mixed as a liquid composition and then dried via, for example, various known spray drying techniques. Spray drying is described as when a liquid form of a composition is sprayed as a mist into a hot, dry camber wherein the aqueous portion of the mist is evaporated by the dry heat of the camber leaving only the dry constituents of the composition in a powdered form. The powdered form of the composition has a moisture content of between about 0.1 % to 5 %. Toothpowders then rehydrate upon use either by the addition of water (*e.g.*, with water applied by the user) at the time of use or by the user's saliva.

Denture cleanser compositions of the invention can additionally include one or more cleaning agents, organic peroxyacid precursors, effervescence generators, chelating agents, etc.

## Mouthwash and Mouth rinse

Typically, the mouthwashes and rinses of the present invention comprise the actinidin-based whitening composition of the present invention in, for example, a water/glycerin solution and may additionally comprise one or more of flavor, humectant, sweetener, sudsing agent (*e.g.*, sodium lauryl sulfate) and colorant as described above for whitening strips. Mouthwashes can include glycerin at a level of from 0 to 60 %, preferably from 0 to 30% by weight. The pH value of such mouthwash preparations is generally from about 3.5 to about 8.0 and preferably from about 4.0 to about 7.5. A pH below 3.5 would be irritating to the oral cavity and dissolve tooth enamel. A pH greater than 8.0 would result in an unpleasant mouth feel. The oral liquid preparations may also contain surface active agents, *i.e.,* surfactants, in amounts up to about 5.0 % and fluoride-providing compounds in amounts up to about 2.0 % by weight of the preparation.

## Chewing Gum

The actinidin-based chewing gum compositions of the present invention may be in the form of a conventional chewing gum Suitable physical forms include sticks, squares and dragees (*i.e.*, sugar coated gum; *e.g.,* Chiclets™). The chewing gum may also have a liquid-filled center wherein the liquid center contains the actinidin-based whitening compositions (*See, e.g.,* U.S. Patent number 6,280,780). The chewing gum may also be a digestible or dissolvable gum suitable for chewing. A chewing gum is typically retained in the oral cavity for a time sufficient to allow ingredients released to contact substantially all of the dental surfaces and/or oral tissues for purposes of oral activity.

The term "carrier materials" as used herein means any safe and effective additional chewing gum components used in the chewing gum compositions of the present invention. Such materials include elastomers, resins, plasticisers, fats, solvents, waxes, emulsifiers, softeners, bulking agents, sweeteners, absorbents, orally active metallic ions, cationic material, fluoride ion sources, additional anticalculus agents, antimicrobial agents, buffers, whitening agents, alkali metal bicarbonate salts, thickening materials, humectants, water, surfactants, titanium dioxide, flavoring agents, xylitol, coloring agents and mixtures thereof.

The chewing gum of the present invention is manufactured by methods known in the art. For example, the gum base is heated to 45° C to soften. The mixer vessel is maintained at 45° C during entire mixing process and additives as described herein as well as the actinidin-based whitening composition of the present invention are added and mixed to homogeneity. Then the gum mixture is formed into sticks, squares, etc., wrapped and cooled. If desired, the gum is coated in a sugar or sugar-free candy coating.

EXPERIMENTAL

Casein Hydrolysis Assay

The following assay is used to determine the activity of crude actinidin, purified actinidin and the actinidin compositions of the present invention.

Prepare the following reagents (milli-Q or distilled water (or equivalent) is used for all dilutions).

50 mM di-sodium hydrogen phosphate is prepared fresh as follows. Weigh 1.42 g of disodium hydrogen orthophosphate anhydrous into a 250ml beaker (or equivalent). Add 160 ml of milli-Q water. Dissolve in warm waterbath. Allow to cool, make to 200 ml in a volumetric flask using milli-Q water. Mix well.

The substrate is prepared fresh as follows. Weigh 1.2 g of Hammarsten Casein (BDH, 440203H) into a 250 ml beaker (or equivalent). Add 160 ml of 50 mM di-sodium hydrogen phosphate. Heat at 60 - 70 °C for 15 minutes. Allow to cool and adjust to pH 8.0 with 0.1 M NaOH, using a pH meter. Bring volume to 200 ml in a volumetric flask (or equivalent) using milli-Q water. Mix well.

L-Cysteine - EDTA is prepared as follows. Weigh 8.78 g of L-Cysteine HCl and 2.23 g of ETDA into a 1 L measuring cylinder. Add approximately 800 ml of milli-Q water. Adjust the pH to 4.5 using 20 % NaOH initially then 0.1 M NaOH towards the end. Make up to a final volume of 1 L using milli-Q water.

0.11 M trichloroacetic acid (TCA) mixed by standard protocols.

Determine the required dilution as follows. Weigh the required amount of sample (for crude Actinidin a typical mass is 1.5 g-1.6 g) into a 100 ml volumetric flask and record the weight. Dissolve and make up to volume with L-Cysteine/EDTA. Place in an ice cooled ultrasonic bath for 10 minutes and then in the fridge for 50 minutes.

Make up the following dilutions:

| Dilution | Enzyme (ml) | L-Cysteine/EDTA (ml) |
| --- | --- | --- |
| 1500 | 3 | 1.5 |
| 2000 | 2 | 2 |
| 2500 | 2 | 3 |
| 3000 | 1 | 2 |
| 4000 | 1 | 3 |
| 5000 | 1 | 4 |
| 6000 | 1 | 5 |
| 7000 | 1 | 6 |
| 8000 | 1 | 7 |
| 10000 | 1 | 9 |
| Note: Keep sample solutions in the refrigerator when not being used. | | |

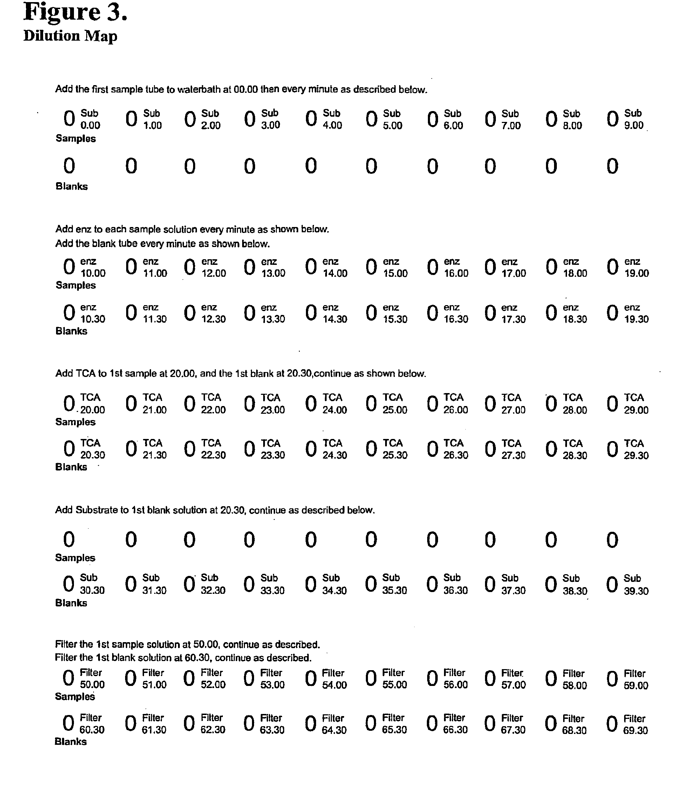

Prepare one sample and one blank test tube (~20 ml) for each dilution. (*See,* Figure 3 for the assay dilution map). Dispense 5 ml of substrate into each of the sample test tubes and 1 ml of enzyme solution into each of the blank test tubes. Place the 1st sample test tube in the 37 °C water bath at 00.00 (minutes.seconds) minutes on the timer. Add one sample tube each minute, at 01.00, 02.00 and so on. At 10.00 minutes, add 1 mL of enzyme reagent to the 1 st sample test tube, mix with vortex mixer for fifteen seconds with brief pulses at 5 and 10 seconds. At 10 minutes, 30 seconds place the first blank (containing enzyme only) test tube into the 37 °C water bath. Add 1 ml enzyme to the next sample solution at 11 minutes, vortex as before. At 11 minutes, 30 seconds add the next blank tube. Continue this pattern with all the sample and blank test tubes. At 20 minutes dispense 5 ml TCA solution to 1st sample test tube and mix with vortex mixer for fifteen seconds with brief pauses at 5 and 10 seconds, then place back in water bath. At 20 minutes, 30 seconds on timer dispense 5 ml of TCA solution into the 1 st blank test tube and vortex for fifteen seconds as above, then place back in the waterbath. At 21 minutes dispense 5 ml TCA into the 2nd sample test tube, vortex for fifteen seconds as above and then place back in waterbath. Continue this pattern with all the sample and blank test tubes. At 30 minutes, 30 seconds dispense 5 ml of substrate to the first blank test tube and vortex for fifteen seconds as above, then place back in the waterbath. Continue this pattern with all of the remaining blank test tubes. Start to filter 1st sample tube at 50 minutes, continue to filter sample tubes one every minute. Use Advantec filter papers #3, 110 mm, for filtration, fold so smooth side of filter paper is out. At 60 minutes, 30 seconds filter the 1st blank solution, at 61 minutes, 30 seconds filter the 2nd blank solution and so on. Stand solutions until they have reached room temperature (15 - 30 minutes). Vortex quickly. Immediately read absorbance at 275 nm; use milli-Q water as blank. Determine from results which dilution has a difference of 0.10 ($\pm$0.01), between the average sample absorbance and the average blank absorbance. Pick the dilution showing a difference of 0.10 $\pm$0.01 to perform the final assay.

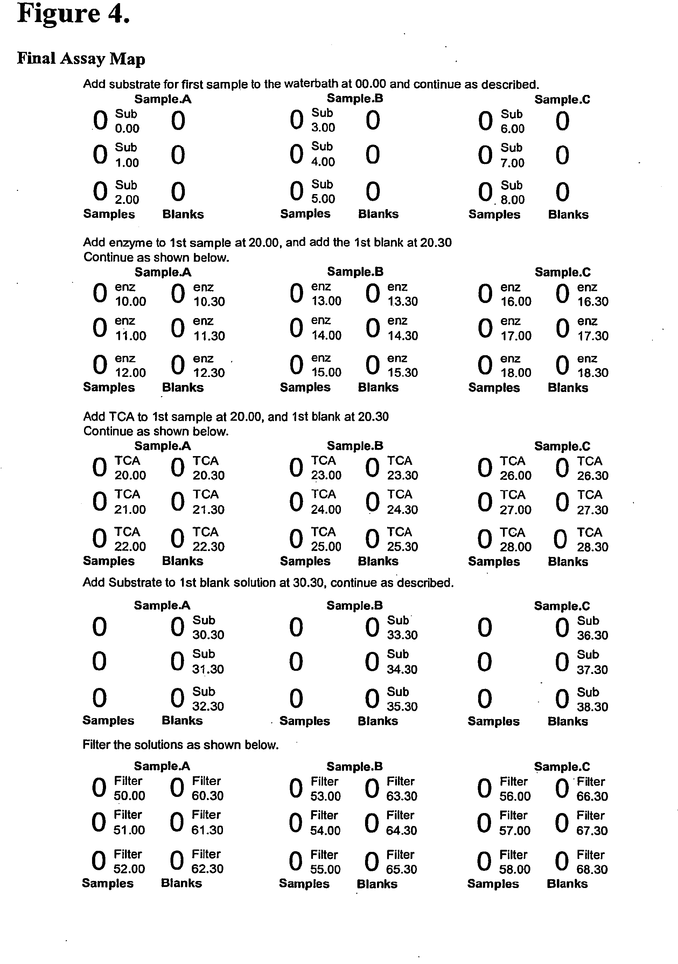

Final Assay - Using determined dilution. Weigh the required amount of sample into a 100 ml volumetric flask and record exact weight. Weigh 65-70 mg of MAC (measurement assurance check based on a batch of actinidin of known activity) into a 100 ml volumetric flask and record exact weight. Top each up to volume with L-Cysteine/EDTA. Place in an ice cooled ultrasonic bath for 10 minutes then in the fridge for 50 minutes. Dilute as appropriate to ensure the absorbance difference between the blank and the samples is 0.10 $\pm$ 0.01 absorbance units using the dilution assay as a guide. A maximum two finished product samples and one MAC sample can be assayed at one time. Prepare three sample and three blank 20 ml test tubes for each solution. (See, Figure 4 for the final assay map). Results are only accepted if the difference between mean sample absorbance and mean blank values is equal to 0.10 $\pm$ 0.01. Calculate the activity as follows:

$$\text{Units}/\text{mg} = \frac{(A10 - A0)}{0.374} \times 50 \times 11 \times \frac{1}{10} \times DF$$

Unit: the production of amino acid equivalent to 1 $\mu$g tyrosine per minute. A10: optical density of the reaction mixture. A0: optical density of the blank mixture. 0.374: optical density of Tyrosine (50 $\mu$g / ml). 50: Tyrosine (50 $\mu$g / ml). 11: reaction volume. 1/10: ten minute reaction time to response in one minute.
Calculate the Dilution Factor (DF) as follows:

$$DF = \frac{100\text{ml}}{\text{mass (mg)}} \times \frac{\text{volume enzyme (ml)} + \text{volume diluent (ml)}}{\text{volume enzyme (ml)}} \times 1000$$

100 ml = Volume actinidin first made to. 1000: Convert from mass in mg to mass in g (as 1 g = 1 ml).

Preparation of Test Samples

In order to evaluate the bioactivity of crude actinidin, a solution made from crude actinidin was measured in a whitening assay using dense ceramic hydroxyapatite disks stained with a solution of tea and coffee. Hydrogen peroxide was used as the positive control and the solvent, water or aqueous glycerin as negative control.

The dense ceramic hydroxyapatite disks (HAD) 1.27 cm diameter $\times$ 0.10 - 0.13 cm thick (0.5" diameter x 0.04-0.05" thick), (Clarkson Chromatography Products Inc., South Williamsport, PA) were rinsed with water twice in order to clean them. The disks were kept in demineralized water for 1 hour. During this time, clarified saliva was prepared by: chewing parafilm (no food or drink 2 hours prior the sampling) and collect saliva in a vial that was then centrifuged at 3500 rpm for 10 minutes. Only the supernatant was used. The disks are removed from water and put in 0.5 ml of clarified saliva and incubated overnight at 37 °C on a platform shaker at 90 rpm.

The next morning, a fresh solution of tea and coffee was prepared. For the tea, 50 ml of boiling water were poured onto 2 Lipton yellow label tea bags in a 100 mL beaker, and left for 10 min. For coffee, 66 g of a regular non-decaffeinated coffee without flavor was used in 1.6 L of spring water.

The saliva was removed from the disks and 0.5 ml of coffee + 0.5 ml of tea were added in the well. The solution and the disks were incubated for 3 hours on a platform shaker at 90 rpm. The tea + coffee solution was removed and the disks were rinsed with water twice and dried with a paper before the L, a, b measurements were taken.

The disks were scanned with a Creoscitex Eversmart Jazz+ scanner using the software Eversmart™ (Creo-Scitex, Billerica, MA). A resolution of about 300 dpi was used to scan and the object was resized with a resolution of 15 dpi. Adobe Photoshop software (Adobe Systems, San Jose, CA). The L, a, b values of the darkest point were taken.

Preparation of Positive Controls

Two hundred microliters of a 30 % peroxide solution were added to 800 $\mu$l of demineralized water (6 % solution). For the 3 % solution, 100 $\mu$l peroxide solution in 900 $\mu$l of demineralized water was used. The solution was mixed and 500 $\mu$l was put directly into 2 wells (positive controls). Two wells were water only added (negative control).

Formulation of Actinidin-Containing Preparation for Application to Teeth

In 3 mL vials, 150 mg of crude actinidin (for a 5 % solution) were weighed and 3 mL of demineralized water were added at room temperature and stirred for 10 minutes at medium speed using a stirring bar. The pH was adjusted to about 6.0 by adding few drops of a 1 N NaOH solution. Five hundred microliters of the crude actinidin solution were added to the disks in the well. Each assay was done in triplicate. The microplate was incubated at 37 °C for 60 minutes and shaken at 90 rpm. The disks were rinsed twice with water. The surface of the HAD was wiped lightly with a paper towel and the color was measured (L*, a* and b* value).

Crude actinidin has a pH optimum of between about 5.0 - 7.0 and is stable at a pH range of between about 4.0 - 8.0. It is also stable below 50 °C for long periods of time.

Quantitative HAD color assessment

The color of the HAD was measured before and after the treatment using the Adobe Photoshop software. After scanning, the value of the optical parameters L*, a* and b* were measured in Adobe. ΔL* represents the difference of L before and after the treatment which relates to the overall lightness or darkness change. Total color difference is given by ΔE* calculated with an equation below. A high value of ΔL* is indicative the lightness and a high value of ΔE is indicative of greater color change. For both values, the higher the value the better the treatment.

$$\Delta E = sqrt(\Delta L^2 + \Delta a^2 + \Delta b^2)$$

where L*:0=black and 100=white

Storage of standards

The positive control ($H_2O_2$) and the ingredients were stored at 4 °C.

RESULTS

Efficacy Experiment

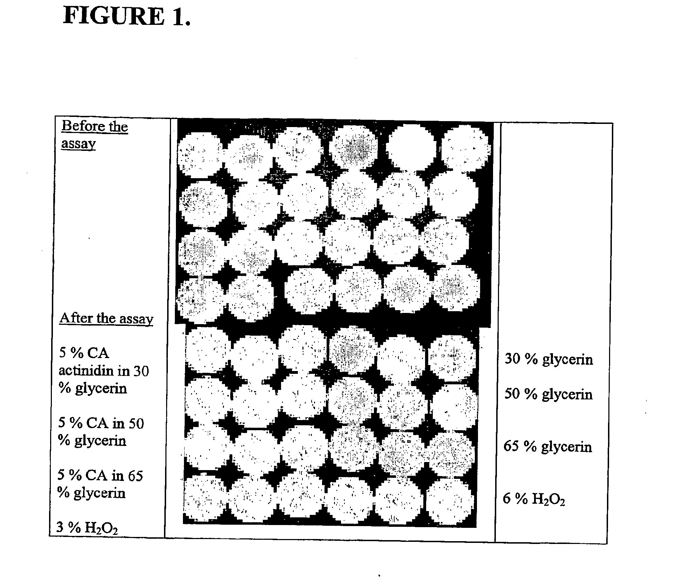

Five percent crude actinidin was able to remove the stains due to coffee and tea (*See,* Table 1, Figure 1). The results were better if the crude actinidin was solubilized in a 50 % glycerin solution where they are comparable to the treatment with a solution of 6 % peroxide. The solution of aqueous glycerin or the water had no effect on the ΔL. Comparable results were obtained using a 5% solution of purified actinidin.

Table 1. Effect of the crude actinidin on the change of lightness and color (ΔL, ΔE), respectively, of hydroxyapatite disks stained with a mixture of tea and coffee compared with peroxide.

| Ingredient | ΔL | Std deviation | ΔE | Std deviation |
|---|---|---|---|---|
| Crude Actinidin (5%) | 10.33 | 2.08 | 15.9 | 1.55 |
| Crude Actinidin (5%) in 50% glycerin | 18.33 | 2.31 | 34.1 | 0.58 |
| Peroxide (3%) | 8.33 | 0.58 | 12.6 | 0.99 |
| Peroxide (6%) | 17.33 | 2.08 | 21.7 | 4.05 |
| 50% glycerin | 1.00 | 1.73 | 5.5 | 2.51 |
| Water | -1.00 | 4.36 | 6.3 | 2.35 |

Time experiment

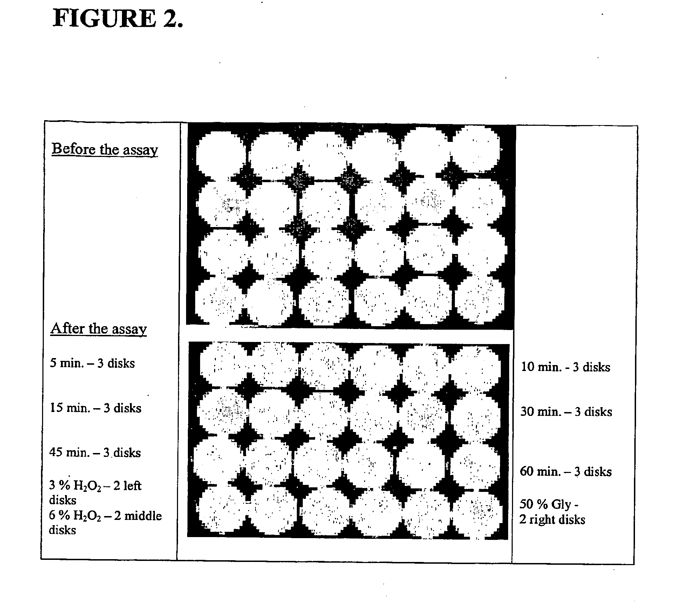

In order to determine the duration necessary to see an effect of crude actinidin, a time experiment was done. The disks stayed in a 5 % crude actinidin solution (50 % aqueous glycerin) for 5, 10, 15, 30, 45 and 60 min. The pH was adjusted to 5.75 with NaOH. Hydrogen peroxide at 3 % and 6 % was used as positive controls and a 50 % glycerin solution was used as a negative control. The results (*See,* Table 2, Figure 2) show that the solution of crude actinidin gave positive results after 5 minutes band after 30 minutes the treatment was comparative to a 3 % peroxide solution. After a 60-minute treatment, the results were superior to a 6 % peroxide solution. The aqueous glycerin solution had no effect on the coloration of the disks.

**Table 2.** Effect of the time on the effectiveness of crude a 5% solution of crude actinidin (CA) to the change of lightness and color ($\Delta L$, $\Delta E$), respectively, of hydroxyapatite disks stained with a mixture of tea and coffee compared with peroxide.

| Ingredient | Time (min.) | $\Delta L$ | Std deviation | $\Delta E$ | std deviation |
|---|---|---|---|---|---|
| CA (5%) in 50% glycerin | 5 | 2.67 | 2.08 | 6.17 | 2.74 |
| CA (5%) in 50% glycerin | 10 | 2.00 | 1.73 | 5.15 | 2.17 |
| CA (5%) in 50% glycerin | 15 | 3.67 | 1.53 | 7.81 | 2.12 |
| CA (5%) in 50% glycerin | 30 | 7.00 | 3.00 | 11.47 | 4.93 |
| CA (5%) in 50% glycerin | 45 | 8.67 | 3.51 | 15.99 | 5.40 |
| CA (5%) in 50% glycerin | 60 | 12.00 | 3.46 | 21.07 | 6.20 |
| Peroxide (3%) | 60 | 7.50 | 0.71 | 11.38 | 0.28 |
| Peroxide (6%) | 60 | 10.00 | 1.40 | 13.76 | 1.85 |
| 50% glycerin | 60 | -1.50 | 0.70 | 3.45 | 2.87 |

**Claims**

1. A peroxide-free method for whitening natural or manufactured teeth, the method comprising:

   a) providing an actinidin-containing preparation;
   b) formulating the actinidin-containing preparation of step a) for application to natural or manufactured teeth; and
   c) contacting the formulation of step b) with natural or manufactured teeth in an amount and for a period of time sufficient to effect whitening of the natural or manufactured teeth;

   wherein the actinidin-containing preparation is formulated in: a whitening strip, a dentifrice; a mouthrinse, a mouthwash; chewing gum; a preparation for soaking manufactured teeth; or a whitening tray.

2. The method of claim 1, wherein the actinidin-containing preparation has a minimum actinidin activity of 50 U/g.

3. The method of claim 1, wherein the actinidin-containing preparation has a minimum final concentration in the preparation of crude actinidin of at least 1.0% w/w.

4. The method of claim 1, wherein the actinidin-containing preparation is formulated in a whitening strip, and the preparation also comprises glycerine at a concentration of 5 to 30% by weight.

5. The method of claim 1, wherein the actinidin-containing preparation is an extract of kiwi fruit.

6. A peroxide-free product for use in a method for whitening natural or manufactured teeth, the product comprising:

   a) a polymeric strip for contact with, for whitening of, natural or manufactured teeth, the polymeric strip being coated with a peroxide-free actinidin-containing preparation; or
   b) a dentifrice comprising a peroxide-free actinidin preparation; or
   c) a whitening tray being coated with a peroxide-free actinidin-containing preparation.

**7.** The product according to claim 6, wherein the actinidin-containing preparation is an extract of kiwi fruit.

**Patentansprüche**

**1.** Peroxidfreies Verfahren zum Aufhellen von natürlichen oder angefertigten Zähnen, bei dem

a) eine Actinidin-haltige Zubereitung bereitgestellt wird,
b) die Actinidin-haltige Zubereitung aus Schritt a) zur Anwendung auf natürlichen oder angefertigten Zähnen formuliert wird und
c) die Formulierung aus Schritt b) in einer ausreichenden Menge und für eine ausreichende Zeitdauer mit natürlichen oder angefertigten Zähnen in Kontakt gebracht wird, um ein Aufhellen der natürlichen oder angefertigten Zähne zu bewirken,

wobei die Actinidin-haltige Zubereitung in einen Aufhellungsstreifen, einer Zahnpasta, einer Mundspülung, einem Mundwasser, Kaugummi, einer Zubereitung zum Eintauchen von angefertigten Zähnen oder einer Bleichschiene formuliert wird.

**2.** Verfahren nach Anspruch 1, bei dem die Actinidin-haltige Zubereitung eine Mindestactinidinaktivität von 50 U/g aufweist.

**3.** Verfahren nach Anspruch 1, bei dem die Actinidin-haltige Zubereitung eine Mindestendkonzentration an rohem Actinidin in der Zubereitung von mindestens 1,0% w/w aufweist.

**4.** Verfahren nach Anspruch 1, bei dem die Actinidin-haltige Zubereitung in einem Aufhellungsstreifen formuliert wird und die Zubereitung auch Glycerin mit einer Konzentration von 5 bis 30 Gew.% enthält.

**5.** Verfahren nach Anspruch 1, bei dem die Actinidin-haltige Zubereitung ein Extrakt der Kiwifrucht ist.

**6.** Peroxidfreies Produkt zur Verwendung in einem Verfahren zum Aufhellen von natürlichen oder gefertigten Zähnen, wobei das Produkt

a) einen polymeren Streifen zum Kontaktieren mit und zum Aufhellen von natürlichen oder gefertigten Zähnen, wobei der polymere Streifen mit einer peroxidfreien Actinidin-haltigen Zubereitung beschichtet ist, oder
b) eine Zahnpasta, die eine peroxidfreie Actinidin-Zubereitung enthält, oder
c) eine Bleichschiene, die mit einer peroxidfreien Actinidin-haltigen Zubereitung beschichtet ist,

umfasst.

**7.** Produkt nach Anspruch 6, bei dem die Actinidin-haltige Zubereitung ein Extrakt der Kiwifrucht ist.

**Revendications**

**1.** Procédé sans peroxyde destiné au blanchiment de dents naturelles ou artificielles, le procédé consistant à :

a) fournir une préparation contenant de l'actinidine ;
b) formuler la préparation contenant de l'actinidine de l'étape a) pour une application à des dents naturelles ou artificielles ; et
c) mettre en contact la formulation de l'étape b) avec des dents naturelles ou artificielles, dans une quantité et pendant une période de temps suffisantes pour réaliser le blanchiment des dents naturelles ou artificielles ;

dans lequel la préparation contenant de l'actinidine est formulée en : une bande de blanchiment, un dentifrice ; un rince-bouche, un bain de bouche ; de la gomme à mâcher ; une préparation destinée à faire tremper des dents artificielles ; ou une gouttière de blanchiment.

**2.** Procédé selon la revendication 1, dans lequel la préparation contenant de l'actinidine présente une activité d'actinidine minimum de 50 U/g.

3. Procédé selon la revendication 1, dans lequel la préparation contenant de l'actinidine possède une concentration finale minimum dans la préparation en actinidine brute d'au moins 1,0 % p/p.

4. Procédé selon la revendication 1, dans lequel la préparation contenant de l'actinidine est formulée en une bande de blanchiment, et la préparation comprend également de la glycérine à une concentration de 5 % à 30 % en poids.

5. Procédé selon la revendication 1, dans lequel la préparation contenant de l'actinidine est un extrait de kiwi.

6. Procédé sans peroxyde destiné à être utilisé dans un procédé de blanchiment de dents naturelles ou artificielles, le produit comprenant :

   a) une bande polymérique destinée à être mise en contact avec des dents naturelles ou artificielles pour le blanchiment de celles-ci, la bande polymérique étant revêtue d'une préparation contenant de l'actinidine sans peroxyde ; ou
   b) un dentifrice comprenant une préparation d'actinidine sans peroxyde ; ou
   c) une gouttière de blanchiment revêtue d'une préparation contenant de l'actinidine sans peroxyde.

7. Produit selon la revendication 6, dans lequel la préparation contenant de l'actinidine est un extrait de kiwi.

# FIGURE 1.

| Before the assay | | 30 % glycerin |
| --- | --- | --- |
| After the assay | | 50 % glycerin |
| 5 % CA actinidin in 30 % glycerin | | 65 % glycerin |
| 5 % CA in 50 % glycerin | | |
| 5 % CA in 65 % glycerin | | 6 % $H_2O_2$ |
| 3 % $H_2O_2$ | | |

# FIGURE 2.

Before the assay

After the assay

5 min. – 3 disks

15 min. – 3 disks

45 min. – 3 disks

3 % $H_2O_2$ – 2 left disks
6 % $H_2O_2$ – 2 middle disks

10 min. - 3 disks

30 min. – 3 disks

60 min. – 3 disks

50 % Gly -
2 right disks

# Figure 3.
**Dilution Map**

Add the first sample tube to waterbath at 00.00 then every minute as described below.

| O Sub 0.00 | O Sub 1.00 | O Sub 2.00 | O Sub 3.00 | O Sub 4.00 | O Sub 5.00 | O Sub 6.00 | O Sub 7.00 | O Sub 8.00 | O Sub 9.00 |

**Samples**

| O | O | O | O | O | O | O | O | O | O |

**Blanks**

Add enz to each sample solution every minute as shown below.
Add the blank tube every minute as shown below.

| O enz 10.00 | O enz 11.00 | O enz 12.00 | O enz 13.00 | O enz 14.00 | O enz 15.00 | O enz 16.00 | O enz 17.00 | O enz 18.00 | O enz 19.00 |

**Samples**

| O enz 10.30 | O enz 11.30 | O enz 12.30 | O enz 13.30 | O enz 14.30 | O enz 15.30 | O enz 16.30 | O enz 17.30 | O enz 18.30 | O enz 19.30 |

**Blanks**

Add TCA to 1st sample at 20.00, and the 1st blank at 20.30, continue as shown below.

| O TCA 20.00 | O TCA 21.00 | O TCA 22.00 | O TCA 23.00 | O TCA 24.00 | O TCA 25.00 | O TCA 26.00 | O TCA 27.00 | O TCA 28.00 | O TCA 29.00 |

**Samples**

| O TCA 20.30 | O TCA 21.30 | O TCA 22.30 | O TCA 23.30 | O TCA 24.30 | O TCA 25.30 | O TCA 26.30 | O TCA 27.30 | O TCA 28.30 | O TCA 29.30 |

**Blanks**

Add Substrate to 1st blank solution at 20.30, continue as described below.

| O | O | O | O | O | O | O | O | O | O |

**Samples**

| O Sub 30.30 | O Sub 31.30 | O Sub 32.30 | O Sub 33.30 | O Sub 34.30 | O Sub 35.30 | O Sub 36.30 | O Sub 37.30 | O Sub 38.30 | O Sub 39.30 |

**Blanks**

Filter the 1st sample solution at 50.00, continue as described.
Filter the 1st blank solution at 60.30, continue as described.

| O Filter 50.00 | O Filter 51.00 | O Filter 52.00 | O Filter 53.00 | O Filter 54.00 | O Filter 55.00 | O Filter 56.00 | O Filter 57.00 | O Filter 58.00 | O Filter 59.00 |

**Samples**

| O Filter 60.30 | O Filter 61.30 | O Filter 62.30 | O Filter 63.30 | O Filter 64.30 | O Filter 65.30 | O Filter 66.30 | O Filter 67.30 | O Filter 68.30 | O Filter 69.30 |

**Blanks**

# Figure 4.

**Final Assay Map**

Add substrate for first sample to the waterbath at 00.00 and continue as described.

|  | **Sample.A** |  |  | **Sample.B** |  |  | **Sample.C** |  |
|---|---|---|---|---|---|---|---|---|
| 0 | Sub 0.00 | 0 | 0 | Sub 3.00 | 0 | 0 | Sub 6.00 | 0 |
| 0 | Sub 1.00 | 0 | 0 | Sub 4.00 | 0 | 0 | Sub 7.00 | 0 |
| 0 | Sub 2.00 | 0 | 0 | Sub 5.00 | 0 | 0 | Sub 8.00 | 0 |
| **Samples** | | **Blanks** | **Samples** | | **Blanks** | **Samples** | | **Blanks** |

Add enzyme to 1st sample at 20.00, and add the 1st blank at 20.30
Continue as shown below.

|  | **Sample.A** |  |  |  | **Sample.B** |  |  |  | **Sample.C** |  |  |
|---|---|---|---|---|---|---|---|---|---|---|---|
| 0 | enz 10.00 | 0 | enz 10.30 | 0 | enz 13.00 | 0 | enz 13.30 | 0 | enz 16.00 | 0 | enz 16.30 |
| 0 | enz 11.00 | 0 | enz 11.30 | 0 | enz 14.00 | 0 | enz 14.30 | 0 | enz 17.00 | 0 | enz 17.30 |
| 0 | enz 12.00 | 0 | enz 12.30 | 0 | enz 15.00 | 0 | enz 15.30 | 0 | enz 18.00 | 0 | enz 18.30 |
| **Samples** | | **Blanks** | | **Samples** | | **Blanks** | | **Samples** | | **Blanks** | |

Add TCA to 1st sample at 20.00, and 1st blank at 20.30
Continue as shown below.

|  | **Sample.A** |  |  |  | **Sample.B** |  |  |  | **Sample.C** |  |  |
|---|---|---|---|---|---|---|---|---|---|---|---|
| 0 | TCA 20.00 | 0 | TCA 20.30 | 0 | TCA 23.00 | 0 | TCA 23.30 | 0 | TCA 26.00 | 0 | TCA 26.30 |
| 0 | TCA 21.00 | 0 | TCA 21.30 | 0 | TCA 24.00 | 0 | TCA 24.30 | 0 | TCA 27.00 | 0 | TCA 27.30 |
| 0 | TCA 22.00 | 0 | TCA 22.30 | 0 | TCA 25.00 | 0 | TCA 25.30 | 0 | TCA 28.00 | 0 | TCA 28.30 |
| **Samples** | | **Blanks** | | **Samples** | | **Blanks** | | **Samples** | | **Blanks** | |

Add Substrate to 1st blank solution at 30.30, continue as described.

|  | **Sample.A** |  |  | **Sample.B** |  |  | **Sample.C** |  |
|---|---|---|---|---|---|---|---|---|
| 0 | 0 | Sub 30.30 | 0 | 0 | Sub 33.30 | 0 | 0 | Sub 36.30 |
| 0 | 0 | Sub 31.30 | 0 | 0 | Sub 34.30 | 0 | 0 | Sub 37.30 |
| 0 | 0 | Sub 32.30 | 0 | 0 | Sub 35.30 | 0 | 0 | Sub 38.30 |
| **Samples** | **Blanks** | | **Samples** | **Blanks** | | **Samples** | **Blanks** | |

Filter the solutions as shown below.

|  | **Sample.A** |  |  |  | **Sample.B** |  |  |  | **Sample.C** |  |  |
|---|---|---|---|---|---|---|---|---|---|---|---|
| 0 | Filter 50.00 | 0 | Filter 60.30 | 0 | Filter 53.00 | 0 | Filter 63.30 | 0 | Filter 56.00 | 0 | Filter 66.30 |
| 0 | Filter 51.00 | 0 | Filter 61.30 | 0 | Filter 54.00 | 0 | Filter 64.30 | 0 | Filter 57.00 | 0 | Filter 67.30 |
| 0 | Filter 52.00 | 0 | Filter 62.30 | 0 | Filter 55.00 | 0 | Filter 65.30 | 0 | Filter 58.00 | 0 | Filter 68.30 |
| **Samples** | | **Blanks** | | **Samples** | | **Blanks** | | **Samples** | | **Blanks** | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5891453 A
- US 5879691 A
- US 6331249 B
- US 6331291 B1
- US 6949240 B
- US 6514483 B
- US 5702251 A
- US 6861048 B
- US 6555094 B
- US 6004538 A
- US 6926916 B
- US 6416774 B
- US 4146634 A
- US 3810874 A
- US 4472480 A
- US 4567073 A
- US 4614667 A
- US 4830910 A
- US 5306758 A
- US 6280780 B

### Non-patent literature cited in the description

- Molecular Clowning: A Laboratory Manual. Cold Spring Harbor Press, 1989
- Protein Purification Protocols. SmithKline Beecham Pharmaceuticals. 2003
- **KING et al.** *Meat Science,* 1981, vol. 5, 389-396
- **LEWIS et al.** *J. Food Biochem,* 1988, vol. 12, 147-158
- **WADA et al.** *Food Chemistry,* 2002, vol. 78, 167-171
- **KOWLESSUR et al.** *Biochem. J,* 1989, vol. 259, 443-452
- **LACHANCE.** *J Am Coll Nutr,* 1997, vol. 16, 5
- **WATTS ; ADDY.** *Food Chemistry,* 2001, vol. 78, 167-171
- **NATHOO.** *J Am Dent Assoc,* 1997, vol. 128, 6S-10S
- **KAUFFMAN ; KELLER.** *Arch Oral Biol.,* 1979, vol. 24, 249-256
- **HAGERMAN ; BUTLER.** *J Biol Chem,* 1981, vol. 256, 494-4497
- **PROCTOR et al.** *J Dental Research,* 2005, vol. 84, 73-78
- **BRAVO.** *Nutrit Rev,* 1999, vol. 56, 317-333
- **BENNICK.** *Crit Rev Oral Biol Med,* 2002, vol. 13, 184-196
- **JOINER et al.** *Eur J Oral Sci.,* 2003, vol. 111, 417-22
- Kirk-Othmer Encyclopedia of Chemical Technology. vol. 21, 207-218